# EUROPEAN PATENT APPLICATION

(11) **EP 3 888 664 A1**
(43) Date of publication of application: **06.10.2021**
(21) Application number: 19917174.5
(22) Date of filing: 28.02.2019
(51) Int. Cl.: A61K 31/728, A61K 38/14, A23L 33/10

(54) **COMPOSITION, FOR PREVENTING, RELIEVING OR TREATING CARTILAGE-RELATED DISEASES OR SYMPTOMS, COMPRISING HAPLN1**

(71) Applicant: HAPLNSCIENCE INC., Gyeonggi-do 13494 (KR)
(72) Inventor: KIM, Dae Kyong, Seongnam-si Gyeonggi-do 13547 (KR); JANG, Ji Min, Seoul 07435 (KR)
(74) Representative: Mewburn Ellis LLP
(86) International application number: PCT/KR2019/002418
(87) International publication number: WO 2020/175721

(57) **Abstract**

The present disclosure relates to a composition for the prevention, improvement, or treatment of cartilage-related diseases or symptoms, the composition including hyaluronan and proteoglycan link protein 1 (HAPLN1) as an active ingredient. In detail, the present disclosure provides a composition for regenerating cartilage, a composition for the prevention, improvement, or treatment of osteoarthritis, or a composition for regenerating growth plate cartilage, each composition including HAPLN1 as an active ingredient.

## Description

### TECHNICAL FIELD

The present disclosure relates to a composition for preventing, improving, or treating cartilage-related diseases or symptoms, the composition including hyaluronan and proteoglycan link protein 1 (HAPLN1) as an active ingredient. Specifically, the present disclosure relates to a composition for regenerating cartilage, or a composition for preventing, improving, or treating osteoarthritis, the compositions including HAPLN1 as an active ingredient. The present invention also relates to a composition for proliferating growth plate cartilage or promoting bone-length growth, or preventing, improving, or preventing short stature.

### BACKGROUND ART

Osteoarthritis is degenerative joint disease which causes pain of joints, swelling of joints, stiffness, stimulates reduced range of motion, and causes destruction of joint cartilage and major bones. Osteoarthritis is known to occur in at least one of ten adults (Lawrence, Felson et al. 2008) and significantly deteriorates patient's quality of life due to pain associated with joint movement. Previously, osteoarthritis was regarded as a general result of aging. However, at present, osteoarthritis is known to be caused by complex interactions among multiple factors including integrity of joints, genetic predisposition, topical inflammation, mechanical force, and cellular and biochemical processes. Pathogenic features thereof include hypertrophy of the lower cartilage, formation of osteoporosis (bone protrusion), and degeneration of the extracellular matrix (ECM) of the articular cartilage that fully fills the joint.

The cartilage thickness in the knee joint is about 2 mm. When an area of about 1 mm² to about 4 mm² is damaged by trauma or a disease, regeneration by natural healing is possible. When an area of about 20 mm² is damaged, self-regeneration is difficult, and generally, great pain is involved. Further, when articular cartilage is completely lost due to various causes such as tumors, necrosis, etc., treatment such as embedding of an artificial joint in the corresponding opening is performed to restore the joint function. However, artificial joints are merely those artificially constructed similarly to joint functions. Since artificial joints are foreign substances in an organism, it is difficult to maintain biocompatibility. In addition, artificial joints are difficult to maintain for more than 20 years because of the complicated operation required under a strict environment of an organism. Deterioration of a resin or metal used as a material thereof, generation of wear debris, or the like may cause a reduction in the function or may cause pain. Further, artificial joints may not be sufficient in durability. Therefore, as a substitute for artificial joint treatment, there is a demand for a technique to regenerate articular cartilage itself.

In addition, recent studies have reported regeneration of articular cartilage by perforating the joint surface and placing collagen containing bone morphogenetic protein (BMP) at a desired site. However, the regenerated articular cartilage is not continuously formed with the neighboring existent articular cartilage, and thus it may not be perfect regeneration. Furthermore, application of collagen to an organism is likely to be avoided, due to problems such as bovine spongiform encephalopathy (BSE), so called mad cow disease, etc. Accordingly, there is a need for the development of a new composition for regenerating cartilage using only materials of which biomedical applications are approved.

Meanwhile, height growth occurs when bone length increases, and endochondral ossification in cartilage is involved in the growth of bone length. Endochondral ossification in cartilage is a process that cartilage is formed and the core of ossification appears in the center thereof to form bones, occurs in epiphyseal plate, and is associated with bone-length growth. To date, growth hormone injection is mainly used as a drug that can promote height growth.

However, the effect is not significant even though growth hormone directly promotes proliferation of chondrocytes of the growth plate. Insulin-like growth factor 1 (IGF-1), secreted when the growth hormone acts on the liver, is a major factor for proliferating chondrocytes. Accordingly, when the concentration of IGF-1 is increased in blood when the growth hormone is injected, it is determined to work normally, and, when growth hormone or IGF-1 acts on chondrocytes, growth factors are secreted and the growth plate is proliferated. Moreover, growth hormone injection is not proper to a child, whose secretion of the hormone is normal, and when used, causes side effects such as hypothyroidism or hyperpigmentation. Addition, the growth hormone injection is expensive. Therefore, there is a need to develop a substance that is capable of inducing height growth by promoting proliferation of growth plate cartilage.

Articular cartilage and growth plate cartilage correspond to hyaline cartilage among various forms of cartilage. The extracellular matrix (ECM) of these cartilage tissues may have an aggregate structure including, as major components, type II collagen, aggrecan, hyaluronan, hyaluronan and proteoglycan link protein 1 (HAPLN1), and the like. Here, numerous aggrecans bind to a hyaluronan chain, and HAPLN1 is known to play a role in physically and chemically stabilizing aggregates by more strongly binding aggrecans to the hyaluronan chain. However, it is not known at all that HAPLN1 has the effect of treating, preventing or improving cartilage-related diseases or symptoms.

Meanwhile, as a study related to cartilage-related diseases or symptoms, US Patent Publication No. 2016/0220699 discloses a method for generating chondrocytes or cartilage-type cells, comprising a step of delivering an expression vector encoding one or more therapeutic polynucleotides to a joint of an individual. Moreover, International Patent Application Publication No. 2017/123951 discloses a method for generating chondrocytes or similar cells thereof, comprising a step of providing an ingredient, derived from nucleus pulposus, to a patient with degenerative disc disease. However, the documents above do not specifically describe the prevention, improvement, or treatment of cartilage-related diseases or symptoms using HAPLN1.

### [Prior Art Documents]

### [Patent Documents]

(Patent Document 0001) US Patent Publication No. 2016/0220699 (published on August 4, 2016)
(Patent Document 0002) International Patent Publication No. 2017/123951 (Published on July 20, 2017)

### DESCRIPTION OF EMBODIMENTS

### TECHNICAL PROBLEM

The present disclosure provides a composition for the prevention, improvement, or treatment of cartilage-related diseases or symptoms using HAPLN1.

Specifically, the present disclosure provides a composition for regenerating cartilage, or a composition for the prevention, improvement, or treatment of osteoarthritis. The present disclosure also relates to a composition for proliferating growth plate cartilage or promoting bone-length growth, or a composition for the prevention, improvement, or treatment of short stature.

### SOLUTION TO PROBLEM

To achieve the above objects, the present disclosure provides a pharmaceutical composition for regenerating cartilage, a food composition for regenerating cartilage, or an animal feed composition for regenerating cartilage, each composition including hyaluronan and proteoglycan link protein 1 (HAPLN1) as an active ingredient.

The present disclosure provides a pharmaceutical composition, and a food composition or animal feed composition for the prevention, improvement, or treatment of osteoarthritis, each including HAPLN1 as an active ingredient.

The present disclosure provides a pharmaceutical composition for proliferating growth plate cartilage or promoting bone length growth, the pharmaceutical composition including HAPLN1 as an active ingredient. The present disclosure also provides a food composition for proliferating growth plate cartilage and promoting bone length growth, the food composition including HAPLN1 as an active ingredient. The present disclosure also provides an animal feed composition for proliferating growth plate cartilage and promoting bone length growth, the animal feed composition including HAPLN1 as an active ingredient.

The present disclosure provides a pharmaceutical composition for the treatment or prevention of bone-length growth disorders, the pharmaceutical composition including HAPLN1 as an active ingredient. The bone-length growth disorders of the present disclosure may be short stature, dwarfism, nanism or precocious puberty.

### ADVANTAGEOUS EFFECTS OF DISCLOSURE

According to the present disclosure, HAPLN1 protein may have cartilage formation-stimulating ability and articular cartilage regeneration ability, and may increase a TGF-β receptor I protein expression level of chondrocytes to increase the population of cells having cartilage formation ability and to induce generation of cartilage tissues. Accordingly, the HAPLN1 protein of the present disclosure, which is a novel material regulating TGF-β signaling, may be usefully applied as a pharmaceutical composition for regenerating cartilage, a food composition for regenerating cartilage, or an animal feed composition for regenerating cartilage.

In addition, the present disclosure provides a composition for the prevention, improvement, or treatment of osteoarthritis by using HAPLN1 protein.

In addition, since HAPLN1 protein proliferates the growth plate cartilage and promotes bone length growth, a height growth therapeutic agent and an adjuvant can be provided with less side effects and better effects than conventional hormones.

### BRIEF DESCRIPTION OF DRAWINGS

FIG. 1 shows the results of SDS-PAGE analysis of the recombinant human HAPLN1 protein obtained using Expi™ 293 cells as a protein expression system.
FIG. 2 shows cartilage formation ability by repeated intraperitoneal administration of HAPLN1 protein into degenerated growth plate of old mice, in which
FIG. 2A shows proteoglycan in tissues, as visualized by safranin O/Fast Green FCF staining, and FIG. 2B shows the presence of chondrocytes having cartilage formation ability, as visualized by immunohistochemistry.
FIG. 3 shows cartilage regeneration ability of HAPLN1 protein intraarticularly administered into damaged knee joint tissues of mice, as visualized by immunofluorescence.
FIG. 4 shows cartilage formation-stimulating ability of HAPLN1 protein for human articular chondrocytes, in which FIG. 4A shows gene expression levels of SOX9, which is a cartilage-specific gene, and aggrecan and type II collagen, which are cartilage matrix components, as determined by a polymerase chain reaction (PCR), and FIG. 4B shows proteoglycan accumulated in extracellular matrix, as visualized by safranin O/Fast Green FCF staining.
FIG. 5 shows TGF-β signaling regulation of the HAPLN1 protein for murine articular chondrocytes, in which FIG. 5A shows TGF-β signaling regulation ability of HAPLN1 protein, as examined by western blotting, FIGS. 5B and 5C show TGF-β receptor I stabilization of HAPLN1 protein, as examined by a PCR and western blotting, and FIG. 5D shows improved cell surface presentation of TGF-β receptor I by HAPLN1 protein, as examined by western blotting.
FIG. 6 shows a schematic diagram summarizing the six mice experimental groups used to analyze osteoarthritis improvement of the HAPLN1 protein.
FIG. 7 shows the articular cartilage tissue morphology improvement of HAPLN1 protein in osteoarthritis-induced mouse, in which FIG. 7A shows the articular cartilage tissue morphology in each mouse experimental group which is confirmed by Safranin O/Fast Green FCF staining, and FIG. 7B shows the quantification of osteoarthritis improvement of HAPLN1 protein obtained by osteoarthritis histopathological evaluation (OARSI score).

### BEST MODE

The present inventors of the present disclosure confirmed cartilage formation-stimulating ability and cartilage regeneration ability of proteoglycan link protein 1 (HAPLN1) protein in old mice and articular cartilage-damaged mice. In addition, the cartilage formation promoting ability and cartilage regeneration ability of the HAPLN1 protein were confirmed to be excellent in chondrocytes. In addition, it was identified that the HAPLN1 protein selectively increased the TGF-β receptor I protein of chondrocyte, thereby completing the present disclosure.

The present disclosure provides a pharmaceutical composition for regenerating cartilage including a HAPLN1 protein as an active ingredient. Preferably, the HAPLN1 protein may promote cartilage formation and may protect articular cartilage. More preferably, the HAPLN1 protein may selectively, stably increase a TGF-β receptor I protein to increase the population of cells having cartilage formation ability and to induce generation of cartilage tissues.

In one embodiment, the present disclosure provides a method of regenerating cartilage in a subject in need of cartilage regeneration, the method including administering an effective amount of HAPLN1 protein to the subject. The term "subject" as used herein includes humans and non-human animals. Non-human animals include all vertebrates, such as mammals and non-mammals, such as non-human primates, sheep, dogs, cattle, horses, and the like.

In one embodiment, the present disclosure provides the use of the HAPLN1 protein for regenerating cartilage.

The present disclosure provides a food or animal feed composition for regenerating cartilage, including HAPLN1 protein as an active ingredient.

The present disclosure provides a pharmaceutical composition for the prevention, improvement, or treatment of osteoarthritis, the pharmaceutical composition including HAPLN1 protein as an active ingredient.

The term "osteoarthritis" used herein is a disease called degenerative arthritis in which the articular cartilage that surrounds the joint surface of the bone is worn down and thus, the bone under the cartilage is exposed and the synovial membrane around the joint is inflamed, resulting in pains and deformation. The term is interpreted as in the sense commonly understood in the art. In appearance, bones called osteophyte grow at the edges of the bones, causing the nodes to stick out and deformed joints. Currently, there is no cure for osteoarthritis, and as pharmacotherapy, painkillers are used to relieve pain, or nonsteroidal anti-inflammatory drugs (NSAIDs) are used to relieve inflammation.

When the HAPLN1 protein according to the present disclosure is used, articular cartilage tissues, in which osteoarthritis develops, are improved to have a morphology similar to normal cartilage. Furthermore, the use of the HAPLN1 protein reduces the degree of damage to the joint surface and reduces the number of fibrosis and osteophyte, thereby preventing, inhibiting or improving the progression of osteoarthritis.

In one embodiment, the present disclosure provides a method of preventing, improving, or treating osteoarthritis in a subject in need of the prevention, improvement, or treatment of osteoarthritis, the method including administering an effective amount of HAPLN1 protein to the subject.

In one embodiment, the present disclosure provides the use of the HAPLN1 protein for the prevention, improvement, or treatment of osteoarthritis.

The present disclosure provides a food or animal feed composition for the prevention or improvement of osteoarthritis, including HAPLN1 protein as an active ingredient.

The present disclosure provides a pharmaceutical composition for proliferating growth plate cartilage or promoting bone length growth, the pharmaceutical composition including HAPLN1 protein as an active ingredient.

The HAPLN1 protein of the present disclosure promotes cartilage formation in the growth plate to increase growth plate activity and strengthen the growth plate. Therefore, the HAPLN1 protein can also be used to treat or prevent bone-length growth disorders. In this case, the bone-length growth disorders include, but are not limited to, short stature, dwarfism, nanism, or precocious puberty.

In one embodiment, the present disclosure provides a method of proliferating growth plate cartilage or promoting bone length growth in a subject in need of proliferating growth plate cartilage and promoting bone length growth, the method including administering an effective amount of HAPLN1 protein to the subject. The present disclosure also provides a method of treating or preventing bone-length growth disorders in a subject in need of treating or preventing bone-length growth disorders, including administering an effective amount of HAPLN1 protein to the subject.

In one embodiment, the present disclosure provides the use of HAPLN1 protein to porliferate growth plate cartilage or promote bone length growth. The present disclosure also provides the use of the HAPLN1 protein for the treatment or prevention of bone length growth disorders.

The present disclosure provides a food or animal feed composition for proliferating growth plate cartilage and promoting bone length growth, the food or animal feed composition including HAPLN1 protein as an active ingredient.

The present disclosure provides a method of regenerating cartilage tissues by treating chondrocytes with HAPLN1. Specifically, provided is a method of regenerating cartilage tissues in vitro using HAPLN1, the method including: isolating chondrocytes from a subject; and treating the chondrocytes with HAPLN1.

When the composition of the present disclosure is a pharmaceutical composition, the composition may include, for administration, a pharmaceutically acceptable carrier, excipient, or diluent, in addition to the above-described active ingredient.

When the composition of the present disclosure is a food composition, the composition may include a variety of nutrients, vitamins, minerals (electrolytes), a flavoring agent such as synthetic and natural flavoring agents, etc., a colorant and a filler (cheese, chocolate, etc.), pectic acid or salts thereof, alginic acid or salts thereof, an organic acid, a protective colloidal thickening agent, a pH modifier, a stabilizer, a preservative, glycerin, alcohols, a carbonating agent used in carbonated beverages, etc.

Hereinafter, the present disclosure will be described in more detail with reference to exemplary embodiments. These exemplary embodiments are only for illustrating the present disclosure in more detail, and it will be apparent to those skilled in the art that the scope of the present disclosure is not limited to these exemplary embodiments in accordance with the gist of the present disclosure.

### Preparation Example: Preparation of HAPLN1 Protein

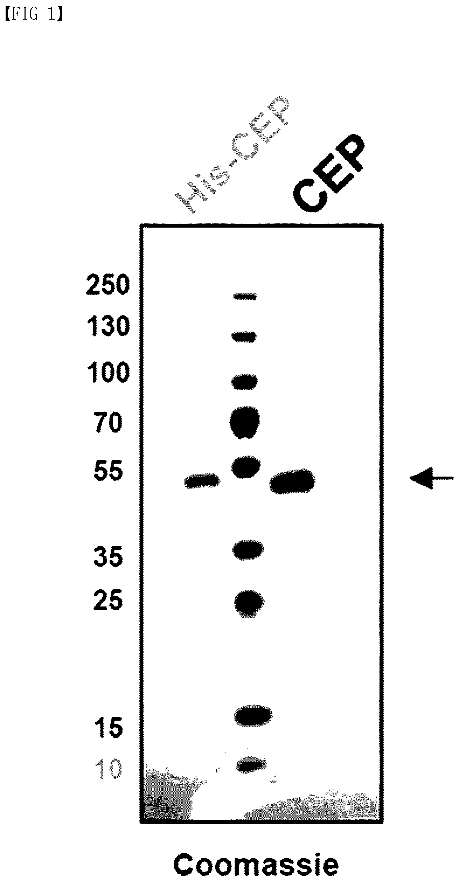

To facilitate protein purification, genes encoding recombinant human HAPLN1 conjugating 10 histidines were synthesized, inserted into plasmid pcDNA3.4-TOPO, and transfected into Expi™ 293 cells using a protein expression system. After 3 days of culture, the cell culture solution was collected and, to purify the protein, passed through a HisTrap column, which is a chelating column, and then, eluted by a concentration gradient of 20 mM to 500 mM imidazole salt using an imidazole PBS buffer containing 0.5 M NaCl. Thereafter, to remove the conjugated histidine, the treatment with TEV protease was performed, and then, the added TEV protease was removed using DynaBeads. The resulting HAPLN1 protein was loaded on SDS-PAGE to confirm the purified protein (FIG. 1). Then, the degree of purification was confirmed to obtain the protein having the purity of 98% or more, and the obtained protein was used in vitro cell experiments or in vivo efficacy test.

Example 1: Analysis of cartilage regeneration ability of HAPLN1 protein in degenerated cartilage tissue in vivo

### 1-1. Stimulation of cartilage formation in degenerated growth plate by repeated intraperitoneal administration of HAPLN1 protein

6-week-old male C57BL/6 mice were classified as a young group, and 20-month-old C57BL/6 mice were classified as an old group. The old group was intraperitoneally administered with HAPLN1 protein diluted with phosphate buffered saline (PBS) at a dose of 0.1 mg/kg daily for 2 weeks, whereas the control group was intraperitoneally administered with PBS in an equivalent manner.

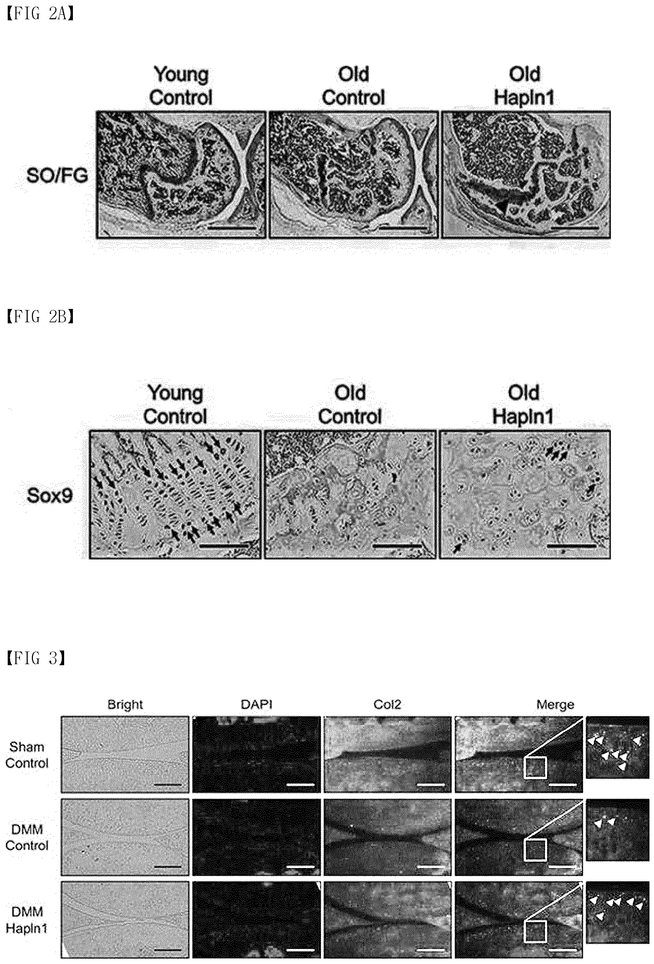

The mouse femur and knee joints of each group were taken and fixed with neutral buffered 10% formalin (NBF) for 48 hours, followed by decalcification with a 10% ethylenediaminetetraacetic acid (EDTA) solution for 7 days. Subsequently, each sample was embedded in paraffin to prepare a paraffin block, and a 5 µm-thick tissue section slide was prepared in a sagittal direction. For histological evaluation, the cartilage tissue of each tissue section slide was visualized by safranin O/fast green FCF (SO/FG) staining. The stained tissue sections were observed and photographed using a Ni-U (Nikon) microscope and DS-Ri1 (Nikon) digital camera, and the results are shown in FIG. 2A (scale bar = 1 mm).

As shown in FIG. 2A, the growth plate of the old control was degenerated and only traces of the cartilage tissue was identified, as compared with that of the young control, while cartilage formation was observed in the degenerated growth plate of the old group (Old HAPLN1), which was repeatedly intraperitoneally administered with HAPLN1 protein (arrow head).

### 1-2. Formation and increase of chondrocytes having cartilage formation ability by repeated intraperitoneal administration of HAPLN1 protein

To identify the presence of cells having the cartilage formation ability at the site of cartilage formation, which was induced by repeated intraperitoneal administration of HAPLN1 protein in Example 1-1, the corresponding site was stained by immunohistochemistry (IHC) using SOX9, which is a cartilage-specific transcription factor. The stained tissue sections were observed and photographed using a Ni-U (Nikon) microscope and DS-Ri1 (Nikon) digital camera, and the results are shown in FIG. 2B (scale bar = 1 mm).

As shown in FIG. 2B, SOX9-expressing cells were retained throughout the cartilage tissue in the young control, whereas no SOX9-expressing cells were found in the old control. However, it was confirmed that a large number of SOX9-expressing cells were found in the cartilage formation-stimulated site of the old group (Old HAPLN1), which was repeatedly intraperitoneally administered with HAPLN1 protein (arrow).

Therefore, it can be seen that the HAPLN1 protein enhances growth plate and promotes bone length growth by proliferating or regenerating the growth plate cartilage.

### Example 2: Analysis of cartilage regeneration ability of HAPLN1 protein in damaged cartilage tissue in vivo

7-week-old male C57BL/6 mice were divided into three groups as follows. A normal control group (sham control group), which is a sham operation group for a destabilization of medial meniscus (DMM) procedure, was bred under the existing conditions for 4 weeks after the procedure. A vehicle treatment group (DMM control group) was bred under the existing conditions for 8 weeks after the DMM procedure, and intraarticularly administered with PBS once a week for the last 4 weeks. A HAPLN1 treatment group (DMM HAPLN1 group) was bred under the existing conditions for 8 weeks after the DMM procedure, and intraarticularly administered with HAPLN1 protein in PBS at a concentration of 1 µg/mL once a week for the last 4 weeks.

At the end of breeding, each knee tissue to which the procedure and treatment were applied was removed and fixed with NBF for 48 hours, and subsequently decalcified with a 10% EDTA solution for 7 days. Subsequently, each sample was embedded in paraffin to prepare a paraffin block, and a 5 µm thick tissue section slide was prepared in a sagittal direction. Type II collagen (Col2) was stained with green fluorescence by immunofluorescence (IF), and nuclei of cells were blue stained with 4',6-diamidino-2-phenylindole (DAPI). The stained tissue sections were observed and photographed using a Ni-U (Nikon) microscope and a DS-Ri1 (Nikon) digital camera, and the results are shown in FIG. 3 (scale bar = 200 µm).

As shown in FIG. 3, it was confirmed that the number of type II collagen-expressing cells found in the normal control group (sham control group) was greatly reduced in the vehicle treatment group (DMM control group), whereas the number of type II collagen-expressing cells was greatly increased in the HAPLN1 treatment group (DMM HAPLN1 group) (arrow head).

Thus, it can be seen that HAPLN1 protein has an excellent effect on cartilage regeneration.

### Example 3: Analysis of cartilage formation-stimulating ability of HAPLN1 protein in vitro

### 3-1. Increase of cartilage formation ability of human articular chondrocytes by HAPLN1 protein

Human articular chondrocytes (HACs) were cultured in a 1:1 mixed medium of Dulbecco's modified Eagle medium/F12 (DMEM/F12; Gibco) containing 10% fetal bovine serum (FBS; Gibco), 1% penicillin/streptomycin (Gibco), and 1% non-essential amino acids (NEAA; Gibco) under conditions of 37°C and 5% CO₂.

As a model for testing the cartilage formation ability of HAC, a three-dimensional culture system in which cells were embedded in alginate beads was used. HAC was uniformly mixed in a 1.25% alginate solution to include 30,000 cells per bead. They were cultured by adding 50 µg/mL of L-ascorbic acid 2-phosphate, 1% insulin-transferrin-selenium (ITS; Gibco), and 10 ng/mL of TGF-β1 to the culture medium. To the HAPLN1 treatment group, 50 ng/mL of HAPLN1 was further added. Culture was continued under conditions of 37°C and 5% CO₂ for 7 days to 28 days.

At the end of the incubation, to recover the HAC embedded in the alginate beads, the alginate was dissolved in 55 mM EDTA solution, followed by centrifugation at 500 x g for 3 minutes. The cells obtained after centrifugation were subjected to RNA extraction and polymerase chain reaction (PCR) to compare and analyze gene expression patterns. Detailed procedures thereof are as follows.

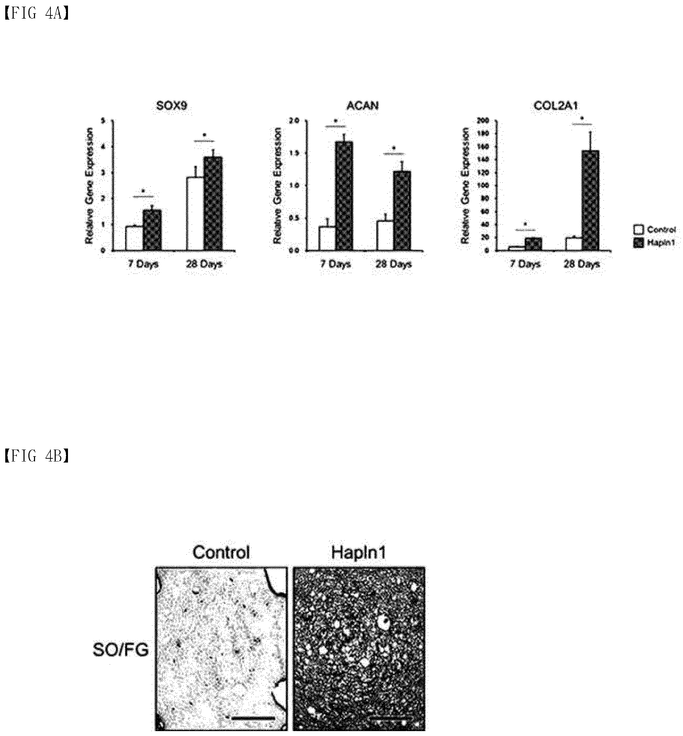

RNA was extracted using a TRIZOL (Thermo Scientific) solution according to the manufacturer's instructions. First-strand cDNAwas synthesized from 0.1 µg of the obtained RNA using oligo-dT20 primers and a SUPERSCRIPT III First-Strand Synthesis Supermix (Invitrogen). The obtained cDNAwas subjected to PCR using 200 nM of primers for each gene of interest and IQ SYBR Green Supermix (Bio-Rad). Reaction conditions included maintaining a temperature at 95°C for the first 5 minutes, followed by 45 cycles having 10 seconds at 95°C, 15 seconds at 62°C, and 20 seconds at 72°C per a cycle. The amplified signal was measured in real-time by CFX CONNECT (Bio-Rad), and an expression level of the gene of interest was calculated as a relative value to each GAPDH expression level. The results are shown in FIG. 4A, and primer sequences used in PCR for each human gene are as follows.

**[Table1]**

| | | |
|---|---|---|
| SOX9 | forward | 5'-AGCGAACGCACATCAAGAC-3' |
| | reverse | 5'-CTGTAGGCGATCTGTTGGGG-3' |
| ACAN | forward | 5'-GTGCCTATCAGGACAAGGTCT-3' |
| | reverse | 5'-GATGCCTTTCACCACGACTTC-3' |
| C0L2A1 | forward | 5'-TGGACGCCATGAAGGTTTTCT-3' |
| | reverse | 5'-TGGGAGCCAGATTGTCATCTC-3' |
| GAPDH | forward | 5'-CTGGGCTACACTGAGCACC-3' |
| | reverse | 5'-AAGTGGTCGrrGAGGGCAATG-3' |

As shown in FIG. 4A, it was confirmed that HAPLN1 protein causes HAC to increase SOX9 gene expression, and at the same time, to increase gene expression of aggrecan (ACAN) and type II collagen (COL2A1).

### 3-2. Increased proteoglycan accumulation in extracellular matrix of human articular chondrocyte by HAPLN1 Protein

From Example 3-1, to evaluate the extracellular accumulation of the cartilage matrix by HAPLN1 addition, the alginate beads at 28 days of culture were fixed with NBF for 15 minutes and frozen in an OCT compound (Sakura) by liquid nitrogen. Thereafter, 5 µm-thick frozen sections were obtained, and after acetone fixation, visualized by safranin O/Fast Green FCF staining. The stained tissue sections were observed and photographed using a Ni-U (Nikon) microscope and DS-Ri1 (Nikon) digital camera, and the results are shown in FIG. 4B (scale bar = 250 µm).

As shown in FIG. 4B, it was confirmed that the accumulation of proteoglycans stained with safranin O was greatly increased in the alginate beads of HAC cultured in the medium containing HAPLN1 protein, as compared with the control group.

### Example 4: Analysis of TGF-β signaling regulation by HAPLN1 protein 4-1. Increased TGF-β receptor I (TβR1) protein in murine articular chondrocyte by HAPLN1 protein

Immature murine articular chondrocytes (iMACs) were isolated from bilateral articular cartilage of 5-day-old ICR mice. The obtained iMACs were cultured in a DMEM/F12 (Gibco) medium containing 10% FBS (Gibco), 1% penicillin/streptomycin (Gibco), and 1% NEAA (Gibco) under conditions of 37°C and 5% CO₂.

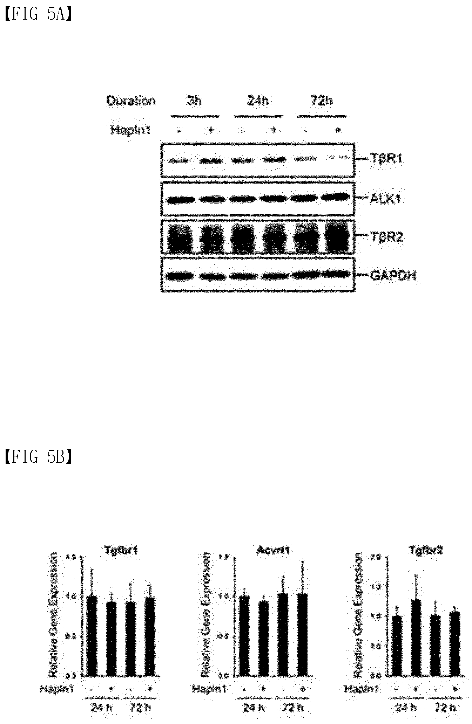

iMACs cultured at a high density on the plate bottom were treated with 100 ng/mL of HAPLN1 for 3 hours to 72 hours, cells were collected, and proteins were extracted in a radioimmunoprecipitation assay (RIPA) buffer. Next, western blotting was performed to examine expression levels of TGF-β receptor I (TβR1), activin receptor-like kinase 1 (ALK1), TGF-β receptor II (TβR2), and Gapdh proteins, and the results are shown in FIG. 5A.

As shown in FIG. 5A, it was confirmed that the protein expression level of TβR1 in iMACs was increased by HAPLN1 protein. In contrast, there were no changes in the ALK1 and TβR2 expression levels.

### 4-2. Increased stability of TβR1 in murine articular chondrocyte by HAPLN1 protein

To demonstrate that the increased TβR1 protein level by HAPLN1 protein as shown in Example 4-1 was attributed to increased stability, gene expression levels of the three proteins, of which levels were compared, were compared and analyzed in the cells cultured for 24 hours and 72 hours under the same experimental conditions, and at the same time, the increase in the TβR1 protein level by HAPLN1 was demonstrated in de novo protein synthesis-limited environment.

RNA extraction and PCR procedure for the analysis are as follows.

RNA was extracted using a TRIZOL (Thermo Scientific) solution according to the manufacturer's instructions. First-strand cDNAwas synthesized from 0.1 µg of the obtained RNA using oligo-dT20 primers and a SUPERSCRIPT III First-Strand Synthesis Supermix (Invitrogen). The obtained cDNAwas subjected to PCR using 200 nM of primers for each gene of interest and IQ SYBR Green Supermix (Bio-Rad). Reaction conditions included maintaining a temperature at 95°C for the first 5 minutes, followed by 45 cycles having 10 seconds at 95°C, 15 seconds at 61°C, and 20 seconds at 72°C per a cycle. The amplified signal was measured in real-time by CFX CONNECT (Bio-Rad), and an expression level of the gene of interest was calculated as a relative value to each GAPDH expression level. The results are shown in FIG. 5B, and primer sequences used in PCR for each mouse gene are as follows.

**[Table 2]**

| | | |
|---|---|---|
| Tgfbr1 | forward | 5'-GTCACTGGAGTTGTACGGCA-3' |
| | reverse | 5'-GGGCTGATCCOGTTGATTIC-3' |
| Acvr11 | forward. | 5'-CTGGGTGCTCTAGGCTTGTG-3' |
| | reverse | 5'-GCCCGTAGTACAGICGCTG-3' |
| Tgfbr2 | forward. | 5'-AACAGTGATGTCATGGCCAG-3' |
| | reverse | 5'-CAGACTTCATGCGGCTTCTC-3' |
| Gapdh | forward | 5'-TGGCCTTCCGTGTTCCTAC-3' |
| | reverse | 5'-GAGTTGCTGTTGAAGTCGCA-3' |

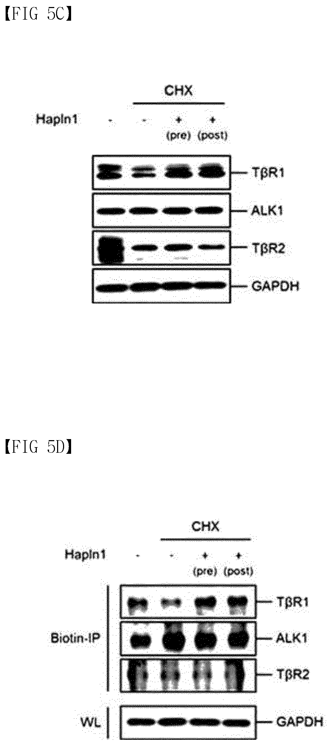

In addition, 200 ng/mL of HAPLN1 was treated from 0.5 hours (pre) before treatment with cycloheximide (CHX) or from 0.5 hours (post) after treatment with cycloheximide (CHX) while iMACs cultured at a high density on the plate bottom were exposed to 10 µM of cycloheximide (CHX).24 hours after the CHX treatment, the cell-attached plate was washed with PBS, cells were collected, and proteins were extracted in a RIPA buffer. The extracted cell lysate was subjected to western blotting to examine protein expression levels of TβR1, ALK1, TβR2, and Gapdh, and the results are shown in FIG. 5C.

As shown in FIGS. 5B and 5C, none of TβR1, ALK1, and TβR2 gene expressions in iMACs was induced or suppressed by HAPLN1 protein. In addition, it was confirmed that TβR1 showed the increased protein expression level by HAPLN1 protein, unlike ALK1 and TβR2, of which protein expression levels were not changed. This suggests that HAPLN1 protein did not induce transcription of the TβR1 gene but increased its half-life, indicating that the stability of the TβR1 protein possessed by iMACs was increased.

### 4-3. Increased cell surface presentation of TβR1 in murine articular chondrocytes by HAPLN1 protein

200 ng/mL of HAPLN1 was treated from 0.5 hours (pre) before treatment with cycloheximide (CHX) or from 0.5 hours (post) after treatment with cycloheximide (CHX) while iMACs cultured at a high density on the plate bottom were exposed to 10 µM of cycloheximide (CHX).24 hours after the CHX treatment, the cell-attached plate was washed with PBS and reacted with EZ-Link Sulfo-NHS-LC-Biotin (Thermo Scientific) for 2 hours to label the cell surface protein with biotin. After the reaction was terminated with a 0.1 M glycine solution, cells were collected and proteins were extracted in an NP-40 lysis buffer (Bioworld). Only the biotin-labeled cell surface proteins were selectively extracted from the extracted lysate by immunoprecipitation using a biotin antibody and magnetic beads. A fraction thus obtained was subjected to western blotting to examine the protein expression levels of TβR1, ALK1, TβR2, and Gapdh, and the results are shown in FIG. 5D.

As shown in FIG. 5D, HAPLN1 protein was found to increase the expression level of TβR1 protein presented on the cell surface of iMACs, and at the same time, there were no changes in the ALK1 and TβR2 expression.

Accordingly, it can be seen that HAPLN1 protein selectively and stably increases TβR1 protein.

### Example 5: Analysis of osteoarthritis improvement by HAPLN1 protein administered in vivo

### 5-1. Evaluation of Articular Cartilage Tissue Morphology Improvement of HAPLN1 Protein in Osteoarthritis-induced Mice

In order to analyze the osteoarthritis improvement of HAPLN1 protein, destabilization-of-medial-meniscus (DMM) treated osteoarthritis model was used as a disease model to induce osteoarthritis by cutting medial meniscotibial ligament in mouse knee joint.

Seven-week-old male C57BL/6 mice were divided into six experimental groups and treated as follows.
- No surgery control (NC-4W) was assigned to 6 animals (n=6) and fed for 4 weeks without any operation.
- Sham operation control (SC-4W) was assigned to 6 animals (n=6) and subjected to a sham operation and then fed for 4 weeks.
- The mild osteoarthritis group (DMM 4-week control, DC-4W) was assigned to 6 animals (n=6), and subjected to DMM operation and then fed for 4 weeks.
- The moderate osteoarthritis group (DMM 8-week control, DC-8W) was assigned to 9 animals (n=9), subjected to DMM operation and then fed for 4 weeks, followed by 4-week intra-articular injection (5 µL) of phosphate-buffered saline (PBS) once per week.
- The low dose HAPLN1 test group (DMM low dose HAPLN1, DL-8W) was assigned to 9 animals(n=9), subjected to DMM operation and then fed for 4 weeks, followed by the 4-week intra-articular injection (5 µL) of 0.1 µg/mL of PBS-diluted HAPLN1 protein once per week.
- The high dose HAPLN1 test group (DMM high dose HAPLN1, DH-8W) was assigned to 9 animals(n=9), subjected to DMM operation and then fed for 4 weeks, followed by the 4-week intra-articular injection (5 µL) of 1.0 µg/mL of PBS-diluted HAPLN1 protein once per week.

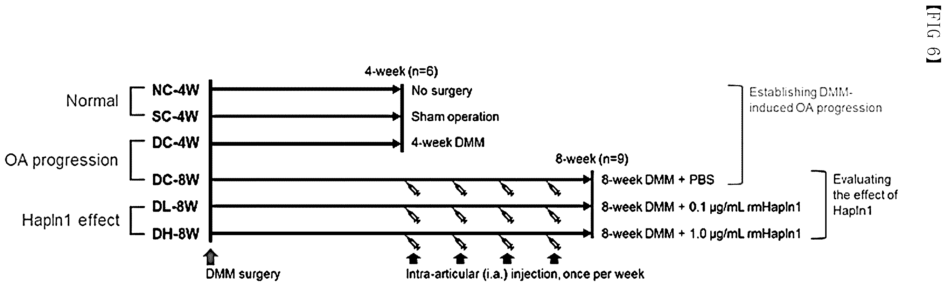

These test groups are summarized in Table 3 and FIG. 6.

**[Table 3]**

| | | | | |
|---|---|---|---|---|
| Group 1 | NC-4W | No surgery normal control | No surgery 4-week normal control | No surgery normal group |
| Group 2 | SC-4W | Sham operation normal control | Sham operation 4-week normal control | Sham operation normal group |
| Group 3 | DC-4W | DMM 4-week control (early OA) | DMM operation 4-week negative control group | Mild osteoarthritis group |
| Group 4 | DC-8W | DMM 8-week control (moderate OA) | DMM operation 8-week negative control group | Moderate osteoarthritis group |
| Group 5 | DL-8W | DMM low dose HAPLN1 | DMM operation 8-week low dose HAPLN1 test group | Low dose HAPLN1 test group |
| Group 6 | DH-8W | DMM high dose HAPLN1 | DMM operation 8-week high dose HAPLN1 test group | High dose HAPLN1 test group |

At the end of feeding, the knee tissue to which the operation and treatment were applied was extracted and fixed with neutral buffered 10% formalin (NBF) for 48 hours, and then, subjected to decalcification with a 10% ethylenediaminetetraacetic acid (EDTA) solution for 7 days. Subsequently, each sample was embedded in paraffin to prepare a paraffin block, and a 5 µm-thick sagittal section slide was prepared.

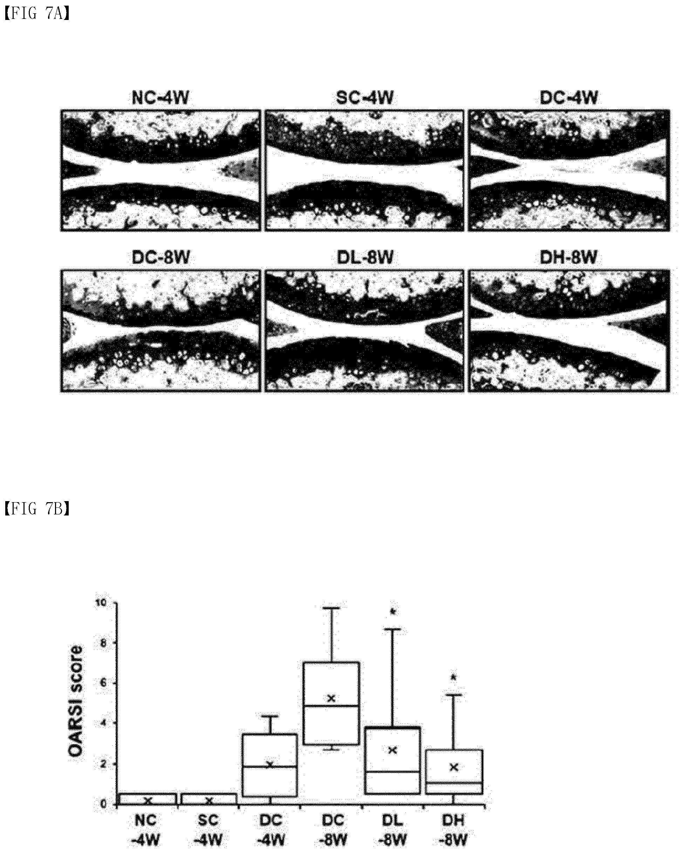

For morphological evaluation of articular cartilage tissue, the cartilage tissue of each tissue section slide was visualized by safranin O/fast green FCF (SO/FG) staining. The stained tissue sections were observed and photographed using a Ni-U (Nikon) microscope and DS-Ri1 (Nikon) digital camera, and the results are shown in FIG. 7A.

As shown in FIG. 7A, in the case of No surgery normal group (NC-4W) and Sham operation normal group (SC-4W), it can be seen that the articular cartilage was intact, and in the case of the mild osteoarthritis group (DC-4W) and the moderate osteoarthritis group (DC-8W), the loss of the joint surface occurred. Meanwhile, in the case of the low dose HAPLN1 test group (DL-8W) and the high dose HAPLN1 test group (DH-8W), the articular cartilage morphology was similar to that of the normal control, and in the case of the high dose HAPLN1 test group (DH-8W), the articular cartilage morphology was almost similar to that of the normal control.

### 5-2. Quantitative Evaluation of Osteoarthritis Improvement of HAPLN1 Protein

In order to quantify the osteoarthritis improvement of the HAPLN1 protein, the tissue stain slide obtained from Example 5-1 was evaluated by osteoarthritis histopathological evaluation (OARSIscore) provided by Osteoarthritis Research Society International (OARSI). This method is used to quantify the degree of damage and fibrosis and osteophyte of the articular surface. The higher the score, the faster the pathology of osteoarthritis.

As shown in FIG. 7B, in the case of No surgery normal group (NC-4W) and Sham operation normal group (SC-4W), there were no symptoms of osteoarthritis, and in the case of the mild osteoarthritis group (DC-4W) and the moderate osteoarthritis group (DC-8W), osteoarthritis progressed in proportion to the feeding period.

However, in the case of the low dose HAPLN1 test group (DL-8W) and the high dose HAPLN1 test group (DH-8W), compared to the moderate osteoarthritis group (DC-8W), which is the negative control with respect thereto, the progression of osteoarthritis was suppressed or osteoarthritis was improved. In addition, it was confirmed that the progression inhibition and improvement of the moderate osteoarthritis group (DC-8W) were returned to the level equivalent to those of the mild osteoarthritis group (DC-4W).

Thus, it can be seen that the HAPLN1 protein is effective for the prevention, improvement, or treatment of osteoarthritis.

## Claims

1. A pharmaceutical composition for regenerating cartilage, the pharmaceutical composition comprising hyaluronan and proteoglycan link protein 1 (HAPLN1) as an active ingredient.

2. The pharmaceutical composition for regenerating cartilage of claim 1, wherein the HAPLN1 stimulates cartilage formation and protects articular cartilage.

3. The pharmaceutical composition for regenerating cartilage of claim 1, wherein the HAPLN1 increases Transforming growth factor beta receptor I (TGF-β receptor I) protein to increase the population of cells having cartilage formation ability and to induce regeneration of cartilage tissues.

4. A food composition for regenerating cartilage, the food composition comprising HAPLN1 as an active ingredient.

5. An animal feed composition for regenerating cartilage, the animal feed composition comprising HAPLN1 as an active ingredient.

6. A pharmaceutical composition for the prevention or treatment of osteoarthritis, the pharmaceutical composition comprising HAPLN1 as an active ingredient.

7. A pharmaceutical composition for proliferating growth plate cartilage or promoting bone length growth, the pharmaceutical composition comprising HAPLN1 as an active ingredient.

8. A pharmaceutical composition for the treatment or prevention of bone-length growth disorders, the pharmaceutical composition comprising HAPLN1 as an active ingredient.

9. The pharmaceutical composition of claim 8, wherein the bone-length growth disorders are short stature, dwarfism, nanism, or precocious puberty.

10. A method of regenerating cartilage tissues using HAPLN1, the method comprising:
isolating chondrocytes from a subject; and
treating the chondrocytes with HAPLN1.
